# EUROPÄISCHE PATENTSCHRIFT

(11) **EP 0 730 739 B1**
(45) Veröffentlichungstag und Bekanntmachung des Hinweises auf die Patenterteilung: **06.02.2002**
(21) Anmeldenummer: 94928791.6
(22) Anmeldetag: 20.09.1994
(51) Int. Cl.: G01N 33/543, G01N 33/552, C12N 11/06

(54) **VERFAHREN ZUR BESCHICHTUNG VON OBERFLÄCHEN MIT BIOMOLEKÜLEN UND ANDEREN REZEPTORMOLEKÜLEN**
PROCESS FOR COATING SURFACES WITH BIOLOGICAL MOLECULES AND OTHER RECEPTOR MOLECULES
PROCEDE D'ENDUCTION DE SURFACES AVEC DES MOLECULES BIOLOGIQUES ET D'AUTRES MOLECULES RECEPTRICES

(30) Priorität: 21.09.1993 DE 4332003
(43) Veröffentlichungstag der Anmeldung: 11.09.1996
(73) Patentinhaber: Molecular Machines & Industries GmbH, 69120 Heidelberg (DE)
(72) Erfinder: Seeger, Stefan, Dr., 93077 Bad Abbach (DE); Hartmann, Andreas, Dr., 68549 Ilvesheim (DE)
(74) Vertreter: Féaux de Lacroix, Stefan, Dr.
(86) Internationale Anmeldenummer: EP9403137
(87) Internationale Veröffentlichungsnummer: WO9508770

(56) Entgegenhaltungen:
- THIN SOLID FILMS (1994), 245(1-2), 206-10 CODEN: THSFAP;ISSN: 0040-6090, 1994 Hartmann, A. et al 'Direct immobilization of antibodies on phthalocyaninato-polysiloxane photopolymers'
- THIN SOLID FILMS, Bd.210/211, Nr.1-2, 30. April 1992, LAUSANNE CH Seiten 710 - 712 I.V. TURKO ET AL. 'Oriented immunoglobulin G layer onto the Langmuir-Blodgett films of protein A' in der Anmeldung erwähnt
- ADV. MATER. (WEINHEIM, FED. REPUB. GER.) (1991), 3(7-8), 388-91 CODEN: ADVMEW;ISSN: 0935-9648, 1991 SCHUHMANN, WOLFGANG ET AL 'Immobilization of enzymes on Langmuir - odgett films via a membrane-bound receptor. Possible applications for amperometric biosensors'
- THIN SOLID FILMS, Bd.210/211, Nr.1-2, 15. April 1992, LAUSANNE CH Seiten 372 - 374 J.P.K. PELTONEN ET AL. 'The polymerization of monolayers of some unsaturated fatty acids' in der Anmeldung erwähnt
- THIN SOLID FILMS (1989), 180, 61-4 CODEN: THSFAP;ISSN: 0040-6090, 1989 OWAKU, K. ET AL 'Preparation and characterization of protein Langmuir - odgett films'
- THIN SOLID FILMS, Bd.216, Nr.1, 16. August 1992, LAUSANNE CH Seiten 105 - 116 GERHARD WEGNER 'Ultrathin films of polymers: architecture, characterization and properties' in der Anmeldung erwähnt
- THIN SOLID FILMS, Bd.152, 1987, LAUSANNE CH Seiten 345 - 376 W.M. REICHERT ET AL. 'Langmuir-Blodgett Films and Black Lipid Membranes in Biospecific Surface-Selective Sensors' in der Anmeldung erwähnt

## Beschreibung

Die vorliegende Erfindung beschreibt eine neuartige Immobilisierungstechnik, mit der DNS, Antikörper. Antigene. Enzyme. Hormone oder andere Peptide kovalent oder nicht kovalent an ein - mittels der Langmuir-Blodgett-Technik auf ein festes hydrophobisiertes Substrat übertragenes - nicht amphiphiles Photopolymer gebunden werden können. Die so dargestellten Schichten können als Meßsonden für Festphasensensoren eingesetzt werden und somit beispielsweise zum quantitativen Nachweis von Biomolekülen, Pestiziden oder sonstiger Analyte dienen.

Zum Nachweis biologischer Substanzen gibt es zahlreiche Methoden, von denen die Immunoassays in der klinischen Chemie die größte Bedeutung erlangt haben. das Prinzip zum Nachweis von Hormonen, Antikörpern oder anderen Proteinen beruht darauf, daß ein an eine Festphase adsorbiertes Rezeptormolekül spezifisch mit den zu bestimmenden Spezies reagiert. Durch den Einsatz einer Markierungstechnik (Fluoreszenzfarbstoff, radioaktive oder enzymatische Markierung) gelingt die quantitative Bestimmung des Analyten über eine Eichmessung [T. Porstmann and S. Kiessig, Journal of Immunological Methods, 150(1992)5]. Ein Nachteil der Immunoassays ist der hohe Zeitaufwand, da bei der Durchfühung der einzelnen Schritte Inkubationszeiten von jeweils bis zu einer Stunde eingehalten werden müssen.

Deshalb ist es in der Praxis von Vorteil eine Meßsonde einzusetzen, welche das Rezeptormolekül (Antikörper, Peptid u.s.w.) bereits in immobilisierter Form enthält, so daß die Anzahl der Präparationsschritte eines Immunoassays für den klinischen Gebrauch verringert wird. Verschiedene Methoden der Immobilisierung von Proteinen sind daher in der Vergangenheit entwickelt worden. Einen Überblick findet man bei H. Weetall [H. Wetall. M. Lynn " immob. enzymes, antibodies, cells and peptides, preparation and charakterisation", M. Dekkar, Inc., New York. (1975) 497]. Die Immobilisierung erfolgt demgemäß meist über die Reaktion einer Aminogruppe des Biomoleküls mit einer Oxofunktion eines Stoffes, der als Verbindungsglied zwischen Oberfläche und zu immobilisierendem Molekül dient. Die bedeutendste Methode stellt hierbei die Silan-Glutaraldehydmethode dar, bei der ein Silan kovalent auf ein zuvor hinsichtlich seiner Hydroxylgruppen aktiviertes festes Substrat fixiert wird. Als Bindeglied zwischen Silanfilm und zu immobilisierendem Biomolekül dient dann Glutaraldehyd. Ein Nachteil herkömmlicher Immobilisierungstechniken liegt in der möglichen Bildung inhomogener globulärer Oberflächenstrukturen [S. Seeger, K Bierbaum, R. Dahint, C. L. Feng, M. Mantar, and M. Grunze: Synthetic Microstructures in Biological Research, J.M. Schnur, M. Peckerar (Hrs.), Plenum Press New York, 53-66, 1992].

Diesen Nachteil vermeidet die Langmuir-Blodgett (LB)-Technik, mit der man ultradünne homogene Filme definierter Schichtdicke auf ein festes Substrat übertragen kann. Auf oder in diesen Filmen lassen sich dann - eventuell nach einer speziellen Aktivierungsreaktion - Biomoleküle immobilisieren. Einen Überblick über LB-Filme und deren Einsatzbereich in der Biosensorik liefert W. M. Reichert [W.M. Reichert. C. J. Bruckner and J. Joseph. Thin Solid Films, 152 (1987) 345]. Der Hauptvorteil der LB-Technik liegt in der Möglichkeit, eine gewünschte Anzahl Monolagen auf das Substrat zu übertragen und durch das Flächenübertragungsverhältnis die Güte des Filmtransfers direkt beim Übertrag zu kontrollieren Verwendet man dann nur die beschichteten Substrate, bei denen der Filmübertrag vollständig ist, so erhält man eine hohe Reproduzierbarkeit der Versuchsergebnisse bei der Durchführung der restlichen Schritte eines Immunoassays. Wendet man die LB-Technik auf Stab-Haar-artige Polymere an, so gelingt der Transfer ultradünner Schichten mit molekular orientiertem Aufbau [E. Orthmann and G. Wegner, Angew. Chemie 98 (1986) 1114]. Ein Beispiel hierfür stellen Polyglutamate mit flexiblen Seitenketten dar, welche befähigt sind, Gastmoleküle in der flüssigen Seitenkettenmatrix aufzunehmen [G. Duda and G. Wegner, Makromol. Chem. Rapid Commun., 9 (1989 495)].

Ein weiterer Nachteil aller bisher geschilderter Methoden ist die fehlende definierte Orientierung der immobilisierten Biomoleküle. Im Falle von immobilisiertem Immunglobulin G ("IgG") ist die Anzahl aktiver Bindungsstellen deutlich geringer als die gesamte. Menge an immobilisierten IgG, da oftmals die Antikörperbindungsstellen durch eine ungünstige Orientierung blockiert sind. Dies äußert sich vor allem in der verringerten Empfindlichkeit eines potentiellen Immunosensors.

Daher sind mehrere Methoden entwickelt worden um Proteine in gezielter Orientierung hinsichtlich der Substratoberfläche zu immobilisieren. V. Turko et al. beschreiben die Stabilisierung einer Vorzugsrichtung von IgG auf einem LB-Trog mit Protein A [I. V. Turko, S. Yurkevich and V. L. Chashchin, Thin Solid Films, 210 (1992) 710]. Außerdem kann man mit der Fromherzmethode ein Protein vom LB-Trog durch Waagerechtes Abstreifen in definierter Orientierung auf ein festes Substrat übertragen [T. Nakagawa and M. Kakimoto, T. Miwa and M. Aizawa, Thin Solid Films, 202 (1991) 151]. Obwohl durch diese Methodik Biomoleküle in hoher Belegungsdichte und definierter Orientierung übertragen werden, ist die praktische Anwendbarkeit dadurch begrenzt, daß die so dargestellten Bioschichten aufgrund fehlender chemischer Bindung zum LB-Film eine schlechte Langzeitstabilität aufweisen. Beim Aufbewahren in Pufferlösung lösen sich die lediglich physikalisch adsorbierten Biomoleküle ab, beim Aufbewahren an Luft erfolgt schon nach kurzer Zeit eine Inaktivierung durch Denaturierung der Proteinstruktur.

Daher wurde bereits nach Möglichkeiten geforscht, Antikörper, Enzyme oder andere Proteine kovalent an den LB-Film zu binden und so eine Stabilitätserhöhung zu erreichen. So veröffentlichte H. Gaub die orientierte Bildung einer Monolage eines F_{ab}-Fragments, das kovalent an ein Phospholipid gebunden ist [M. Egger, S. P. Heyn and H. E. Gaub, Biophys. J., Vol. 57 (1990) 669]. Die Haltbarkeit derartig immobilisierter Biommoleküle ist jedoch dadurch begrenzt, daß sich die nicht vernetzten Phospholipidfilme bei Lagerung in Pufferlösung mitsamt der immobilisierten Bioschicht vom Substrat ablösen.

Ein Hauptnachteil bisheriger Immobilisierungsmethoden liegt demnach in der mangelhaften Langzeitstabilität der Bioschichten, welche auf LB-Filmen immobilisiert wurden.

Die vorliegende und in Anspruch 1 dargestellte Erfindung betrifft ein neues Immobilisierungskonzept mit dem Biomoleküle (Antikörper, Antigene, Enzyme, DNS, Hormone u.a.) kovalent an eine quervernetzbare nicht amphiphile LB-Filmsubstanz gebunden werden können. Ein nicht amphiphiler polymerisierbarer LB-Film wird - entweder vor und/oder nach dem LB-Transfer auf das speziell vorbehandelte Substrat - quervernetzt und danach hinsichtlich Biomolekül bindender Funktionen aktiviert, falls dies nicht schon in einem vorangehenden Schritt geschehen ist, oder Biomolekül bindende Funktionen bereits in der ursprünglichen Filmsubstanz vorhanden sind. Dieser Aktivierungsschritt entfällt auch dann, falls das Biomolekül, zusammen mit der Filmsubstanz auf dem Trog aufgespreitet wurde, so daß es in die Matrix des Photopolymers eingebaut wird. Ansonsten wird das Biomolekül nach einer weiteren eventuellen Vernetzungsperiode immobilisiert, indem es entweder über die LB-Technik auf das Substrat übertragen wird und danach automatisch mit der Filmsubstanz reagiert oder das Substrat in einer Lösung des Biomoleküls inkubiert. Es können auch mehrere LB-Filmsubstanzen und/oder mehrere Biomoleküle kombiniert eingesetzt werden. Weitere bevorzugte Ausführungsformen finden sich in den Unteransprüchen.

Demgemäß besteht die vorliegende Erfindung aus einer neuen Immobilisierungsmethode für Biomoleküle, welche folgendermaßen aufgebaut ist:
A) Übertrag einer oder mehrerer Monolagen einer zwei- oder dreidimensional photochemisch vemetzbaren Filmsubstanz auf ein Substrat. Es können auch verschiedene LB-Filmsubstanzen kombiniert eingesetzt werden. Mindestens eine der beteiligten LB-Substanzen ist dabei photochemisch vernetzbar und nicht amphiphiler Natur, oder aber eine nicht amphiphile Substanz wird erst in Kombination mit den restlichen LB-Filmsubstanzen oder durch Zusatz gewisser Sensibilisatoren photochemisch vernetzbar gemacht. Die Vernetzung der LB-Filme erfolgt entweder vor und/oder nach dem Filmübertrag und entweder vor und/oder nach dem Einbau bzw. der Immobilisierung der Biomoleküle.
B) Einbau der zu immobilisierenden Rezeptor-Biomoleküle oder Partikel (Antikörper. Antikörperfragmente, Enzyme, Hormone, Antigene, DNS, Zellen, Viren, Peptide. ionenselektive Krononether u. a.) in die Polymermatrix entweder vor dem Filmübertrag durch Cospreiten mit der Filmsubstanz und gemeinsamem Aufzug des Substrats, oder durch Immobilisierung auf der Biomolekül bindende Funktionen enthaltenden Filmsubstanz nach dem Übertrag.
C) Eine eventuell notwendige Aktivierung der Filmsubstanz zur Darstellung Biomolekül bindender Funktionen kann mit einem wiederverwertbaren Oxidationsreagens in einer heterogenen Reaktion vom Lemieux Typ erfolgen, so daß olefinische Doppelbindungen in Oxofunktionen überführt werden.
D) Die Immobilisierung des Biomoleküis kann - wenn nicht schon durch Cospreiten mit der Filmsubstanz geschehen - auf kovalente Weise dadurch erfolgen, daß die Biomoleküle entweder per LB Technik oder durch Inkubieren in einer Lösung der Biomoleküle über tragen werden. Es können auch verschiedene Biorezeptoren unterschiedlicher Spezifität auf ein und dasselbe Substrat immobilisiert werden.

Die Immobilisierung von biologischen Rezeptormolekülen auf amphiphilen LB Filmen zur Entwicklung eines optischen Biosensors, der auf der Basis des Fluoreszenz-Energietransfers beruht, wurde von Hugl und Mitarbeitern beschrieben [H. Hugl et al., Deutsches Patentamt: DE 4013713]. Im Unterschied dazu beschreibt die vorliegende Erfindung die Immobilisierung von Biomolekülen auf *nicht*-amphiphilen LB Filmen, die auf ein hydrophobes Substrat aufgezogen werden. Aufgrund des "Stab-Haar-Konzepts" neigen solche LB Filme nicht zur Domänenbildung, wie dies bei amphiphilen Filmen der Fall ist [E. Orthmann, G. Wegner, Angew. Chem., **98** (1986) 1114]. Daher haben die nach der vorliegenden Erfindung hergestellten Filmoberflächen einen homogeneren Aufbau. Auch nach der photochemischen Vernetzung bleibt dieser bis in molekularen Dimensionen erhalten. Als Träger (Substrate) kommen alle bekannten Materialien in Frage, welche für nicht amphiphile Filmsubstanzen geignet sind, z. B. hydrophobisiertes Glas, Quarzglas, Lithiumniobat, Silicium (u. a. Metalle), hydrophobe Kunststoffe, Folien, Membrane oder sonstiges hydrophobes Material. Auch hydrophobisierte Zähne, Knochen, Teile von Organimplantaten können in Frage kommen. Außerdem eignen sich hydrophobisierte Fasern aus Glas, Kunststoff u.s.w.. Zusätzlich zu einer eventuell notwendigen Hydrophobisierung nicht hydrophober Substrate, können diese vorher hinsichtlich hydrophiler Gruppen aktiviert werden. Beispielsweise werden hydrophile Hydroxygruppen durch Wasserstoffperoxid und Salzsäure aktiviert. Ein hierfür geignetes Hydrophobisierungsreagens ist Dimethyldichlorosilan.

Als Filmsubstanzen eignen sich eindimensionale verknüpfte Polymere, welche die Eigenschaft haben, photochemisch vemetzbar zu sein. Bevorzugt eingesetzt werden hierbei nicht amphiphile LB-Filme, da sie als Stab-Haar-Polymere die Möglichkeit der molekularen Orientierung implizieren, und so LB-Filme gezielter Architektur auf das Substrat übertragen werden können [G. Wegner, Thin Solid Films, **216** (1992) 105-116]. Ein Beispiel hierfür ist ein mit Alkylketten substituiertes Phtalocyaninatopolysiloxan-Derivat [E. Orthmann. G.

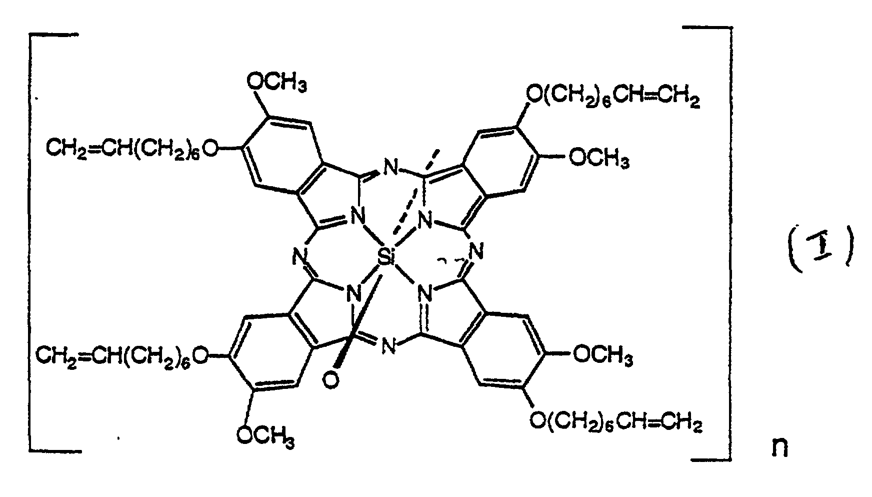

Wegner, Angew. Chem., **98** (1986) 114] mit terminalen olefinischen Doppelbindungen - " Peps" (siehe Formel I).

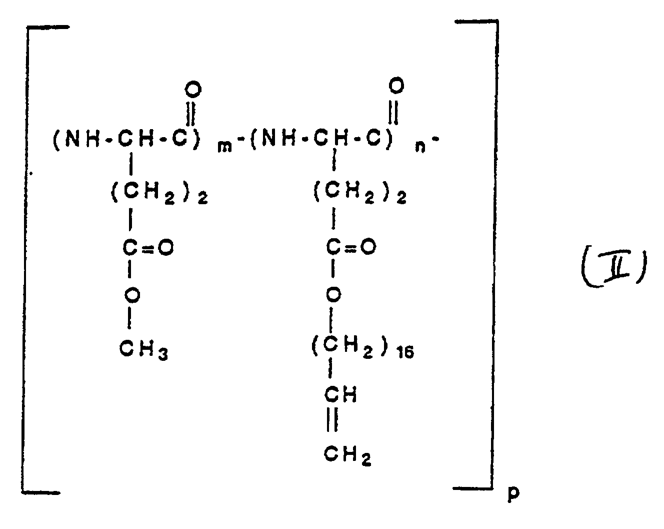

Ein weitere Klasse nicht amphiphiler Filmpolymere stellen Polyglutamate [G. Duda. A. J. Schouten, T. Arndt, G. Lieser, G. F. Schmidt, C. Bubeck and G. Wegner, Thin Solid Films, 159 (1988) 221] dar, welche ebenfalls Stab-Haar-Polymere bilden siehe Formel II).

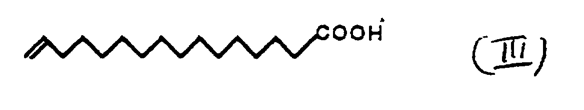

Außerdem kommen die gleichen Substanzklassen in Frage, wenn sie anstelle der olefinischen Endgruppen andere photochemisch quervernetzbare Gruppen enthalten z.B. Carbonyl-. Thionyl-, Imingruppen oder auch Kohlenstoffdreifachbindungen. Außerdem muß sich die vernetzbare Gruppe nicht unbedingt am Ende der Alkylseitenketten befinden. Sie kann sich auch an einer anderen Stelle befinden. Neben nicht amphiphilen Filmen, lassen sich auch solche amphiphiler Natur, zum Beispiel Fettsäuremoleküle mit vernetzbaren olefinischen Doppelbindungen verwenden [J. P. K. Peltonen, H. Pingsheng and J. B. Rosenholm, Thin Solid Films, 210/211 (1992) 372] (siehe Formel III).

Allerdings läßt sich auf diese Art und Weise keine gezielte Molekülarchitektur gemäß dem Stab-Haar-Konzept betreiben.

Weitere einsetzbare Filme sind Cyclodextrine, Polysaccharidfilme, Polysiloxanfilme u. a., welche alle mit Alkylseitenketten substituiert sind und an diesen Substituenten tragen, welche sie polymerisierbar machen.

Die zur Kombination mit den bisher besprochenen LB-Filmsubstanzen geigneten Substanzen sind entweder selbst derartige Filmsubstanzen, oder aber sie fungieren als Sensibilisatoren, die das für die photochemische Vernetzung notwendige Licht absorbieren und auf die vernetzbare Filmsubstanz übertragen. Hierbei kann der Sensibilisator auch selbst vernetzbar sein. Eine geignete Kombination stellt zum Beispiel Copolyglutamat und Pcps dar. Copolyglutamat ist mangels Licht absorbierender Gruppen nicht vernetzbar, läßt sich jedoch unter Anwesenheit von Pcps bei 254 nm oder anderer Sensibilisatoren photochemisch polymerisieren (s. Beispiel 6).

Für den Übertrag der Filme auf das Substrat eignet sich die Langmuir-Blodgett-Technik. Hierbei spielt es keine Rolle, ob das Substrat senkrecht eingetaucht wird, oder der Film durch waagrechtes Abstreifen aufgezogen wird. Als Subphase für die Filmsubstanzen eignet sich idealerweise entionisiertes und mit einer Milliporeanlage gereinigtes Wasser. Aber auch Salz- und Pufferlösungen kommen - je nach eingesetzter Filmsubstanz - in Frage. Der Übertrag findet bei Oberflächenspannungen von 10 bis 50 mN/m statt und bei Temperaturen zwischen 0 °C und 50 °C.

Die Vernetzung der Photopolymere kann bereits vor dem Filmübertrag stattfinden. Hierbei empfiehlt es sich die Filmsubstanz(en) direkt auf dem Trog mit einer UV-Lampe zu bestrahlen. Als Lichtquelle eignet sich prinzipiell jede UV-Lampe.

Dies gilt auch für eine Vernetzung nach dem Filmübertrag. Die Vernetzung von Pcps gelingt mit einer Quecksilberniederdrucklampe der Leistung 30 Watt in 15 cm Entfernung von der Filmsubstanz bei 254 nm. Übliche Bestrahlungsdauern liegen zwischen 5 Minuten und mehreren Stunden. Je länger bestrahlt wird, umso größer wird jedoch die Gefahr einer möglichen Photozerstörung der Filme. Falls das zu immobilisierende Biomolekül oder ein geignetes Derivat mit den Filmsubstanzen auf dem LB-Trog cogespreited wird und vor oder nach dem Filmübertrag die photochemische Vernetzung durchgeführt wird besteht zusätzlich die Gefahr der Denaturierung der Biomoleküle.

Bei der Vernetzungsreaktion reagieren ungesättigte Gruppen miteinander. Die photochemische Polymerisierung von olefinischen Doppelbindungen kann zum einen als [2]+[2]-Addition erfolgen, wodurch bi- oder polyfunktionelle Monomerbausteine quervernetzt werden. Denkbar ist auch eine Radikalkettenpolymerisation. Diese kann zum Beispiel auch über einen Initiator gestartet werden, der UV Licht absorbiert und so die für die Bildung reaktiver Spezies notwendige Energie liefert [N. S. Allen: "Photoinitiators for Ultraviolett Curing", Trends in Polymer Science, Vol. 1, No. 7 (1993) 213]. Hierbei haben sich Acrylgruppen (C=C-C=O) als reaktivste Gruppen für eine photochemische Vernetzung mit Photoinitiatoren erwiesen [N. S. Allen, and M. Edge, J. Oil Colour Chem. Assoc. 73 (1990)438]. Diese haben zudem den Vorteil nach ihrer Vernetzung noch Biomolekül bindende Oxogruppen zur Verfügung zu stellen, so daß eine weitere Aktivierungsreaktion entfällt.

Falls die Filmsubstanzen hinsichtlich Biomolekül bindender Funktionen aktiviert werden müssen, eignet sich die oxidative Spaltung von Kohlenstoffdoppelbindungen mit Osmiumtetroxid und Natriumperjodat nach Art der Lemieuxreaktion. Verbrauchtes Osmiumtetroxid wird dabei während der Reaktion vom überschüssigen Perjodat immer wieder nachgebildet. Die Literatur [R. Pappo, D. S. Allen, R.U. Lemieux, and W. S. Johnson, J. Org. Chem. **21**, 478 (1956)] beschreibt das Arbeiten mit einer Lösung des Olefins in einem Ether-Wasser-Gemisch. Für die Oxidation der Filmsubstanz vor dem Übertrag auf den Träger kann diese Variante direkt übernommen werden. Soll jedoch der LB-Film (mit olefinischen Gruppen) erst nach dem Übertrag auf das Substrat oxidiert werden, so impliziert dies eine andere Reaktionsführung: Hierfür wird auf den Einsatz von Ether verzichtet, der die Filmsubstanz vom Substrat ablösen würde. Die Oxidation der terminalen Doppelbindungen kann demge- 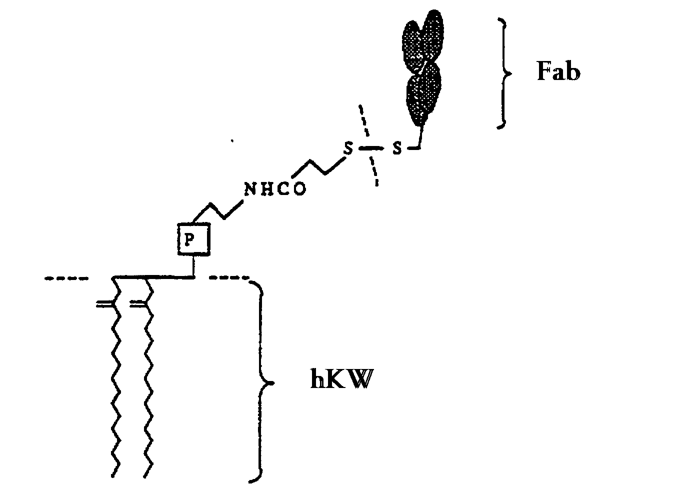 mäß nur an der Grenzfläche zwischen flüssiger Phase (Wasser, Osmiumtetroxid and Natriumperjodat) und fester Phase (festes Substrat und LB-Film als flüssig kristalliuer Phase) stattfinden.

Ein Beispiel für ein immobilisierbares Biomolekül stellt die Klasse der Immunglobuline dar. Immunglobuline zählen zu den Eiweißstoffen und besitzen demgemäß Aminogruppen enthaltende Bausteine. Diese reagieren in bekannter Art und Weise mit Aldehydgruppen der Filmsubstanzen zu Imingruppen. Eine Reduktion der Imingruppen zu Amingruppen führt zu einer zusätzlichen Stabilisierung der Bindung. Noch stabiler sind Amidgruppen, weshalb man auch Estergruppen der Filmsubstanzen mit den Aldeydgruppen reagieren läßt. Dies ist in der Literatur bereits beschrieben worden [H. Wetall, M. Lynn "immob. enzymes, antibodies, cells and peptides, preparation and characterisation", M. Dekkar, Inc., New York. (1975) 497]. Andere Biomoleküle lassen sich - sofern sie den Eiweißen angehören - analog immobilisieren. Wenn die Biomoleküle mit den Filmsubstanzen cogespreitet werden sollen, so müssen sie geeignet substituiert sein. Geignet sind zum Beispiel f_{ab'}-Fragmente des IgG, die an ihren Thiogruppen mit einem längerkettigen Kohlenstoffrest substituiert sind, wie dies bereits Gaub et al. beschrieben haben [M. Egger, S. P. Heyn, and H. E. Gaub: "Two-dimensional recognition pattern of lipid-anchored f_{ab'}- fragments", Biophys. J., VoL 57 (1990) 669]. In dem vorstehenden Formelschema bedeuten P=Phosphat und hKW= hydrophober konlenwasserstoffrest.

Ein derartig substituiertes Biomolekülfragment sollte sich auf dem LB-Trog zusammen mit einer photochemisch vernetzbaren Substanz cospreiten lassen. Vor oder nach dem Übertrag auf das Substrat wird dann der Film vemetzt, wodurch das Biomolekül fixiert wird. Der Vorteil hierbei ist, daß durch die LB-Technik die gewünschte einheitliche Orientierung aller zu immobilisierender Moleküle erreicht wird, was sich in einer erhöhten Bioaktivität und einer damit einhergehenden Erhöhung der Empfindlichkeit eines potentiellen Biosensors äuβert.

### Beispiel 1

Nach verschiedenen Vernetzungszeiten auf dem LB-Trog wurden Schubflächendiagramme angefertigt, mit dem Ergebnis, daß zunehmende Vernetzungszeiten erhöhte Kollapsdrücke zur Folge haben. So liegt der Kollapsdruck nach 87 Minuten Vernetzung bei 45 mN/m (Raumtempeatur). Eine unvernezte Probe hat dagegen einen Kollapsdruck von nur 34 mN/m (Raumtemperatur). Mit zunehmender Bestrahlungsdauer nimmt also der Polymerisationsgrad zu.

Nach eineinhalb Stunden ist der Polymerisationsprozeß noch nicht abgeschlossen. Analoge Untersuchungen am Beispiel eines Fettsäurederivats mit terminalen Kohlenstoffdoppelbindungen wurden von Peltonen et al. durchgeführt [J. P. K. Peltonen, H. Pingsheng and J. B.

Rosenholm. Thin Sol. Fi., 210/211, (1992), 372]. Die entsprechende Vernetzung von auf das Substrat übertragener Filme, ist ebenfalls mit Erfolg durchgeführt worden (s.u.).

### Beispiel 2

Oxidation einer Monolage Pcps auf Glas:
Zu 1,6 ml Wasser (Milipore gereinigt) fügt man 0,9 µl Osmiumtetroxid in tert-Butanol (1 ng/µl) und das Substrat zu und rührt 5 Minuten. Dann gibt man bei Raumtemperatur 0,05g Natriumperjodat zu und läßt ca 2 Stunden - unter gelegentlichem Schütteln - ruhen. Danach wird mit einer Phosphatpufferlösung und Tween-20 (einem Detergens) gespült.

Die so preparierten Substrate zeigen eine hohe Affinität hinsichtlich nucleophiler Aminogruppen von Antikörpern, was mit einem enzyme-linked immuno sorbent assay (ELISA) überprüft wurde (s.u.).

### Beispiel 3

Immobilisierung von Immunglobulin G, Blockierung, ELISA:
Die Immobilisierung von IgG erfolgte gemäß dem Fachmann bekannter Vorschriften:
Die oxidierten Polymerfilme auf Glas werden in eine Lösung von 5 µl Ziege-anti-Kaninchen IgG in 1 ml PBS gestellt und dann ca. eine Stunde darin bei 4° C belassen.

Nach einem kurzen Spülvorgang mit PBS-Tween 20 werden die nicht abreagierten Aldehydfunktionen durch Ethanolamin blockiert, indem man die Proben für 15 Minuten einer Lösung von 50 µl Ethanolamin und 1 ml PBS aussetzt.

Nach dreimaligem Waschen mit PBS-Tween 20 und Spülen erfolgt eine Stunde Inkubation mit 5 µl Kaninchen-anti-Pferd IgG-POD-Konjugat in 1 ml PBS, oder beim Blindwert (Kontrolle der unspezifischen Adsorption von IgG) die gleiche Menge Ziege-anti-Meerschweinchen IgG-POD-Konjugat.

Schließlich wird erneut drei Mal gewaschen und gespült, und das Glasstückchen in eine Küvette mit Rührvertiefung und Mikrorührfisch gestellt, und mit der auf Raumtemperatur befindlichen ELISA-Substratpufferlösung (2,5 ml) gefüllt. Das Glasstückchen befindet sich nicht im Lichtgang des UV-Vis- Photometers. Nun gibt man einen Mikroliter Wasserstoffperoxid zu und registriert alle 30 Sekunden bei 405 nm die Extinktion (vorher Nullabgleich), welche mit der Farbstoffentwicklung zunimmt. Die Steigung der Extinktionsgeraden wird dann mit einer Eichmessung verglichen und so die Menge an aktivem immobilisiertem IgG ermittelt.

Wird IgG auf einem Pcps-Film immobilisiert, der 1 Stunde vernetzt wurde, so erhält man demgemäß eine Belegungsdichte von 0,09 ng/mm². Die unspezifische Adsorption, welche mit einem Blindantikörper getestet wurde beträgt ca. 10 %. Ein Peps-Film, auf dem keine Aldehydgruppen generiert wurden, zeigt demgegenüber eine deutlich geringere Antikörperaffinität: Es ergeben sich Belegungsdichten, die im Bereich der unspezifischen Adsorption liegen.

### Beispiel 4

Abhängigkeit der aktiven Antikörperbelegungsdichten von der Bestrahlungsdauer der Pcps-Filme:

Wenn man 10 Monolagen Pcps auf hydrophobisiertem Glas unterschiedlich lange bestrahlt. und darauf Antikörper (Ziege-anti-Kaninchen IgG) immobilisiert, erhält man unterschiedliche ELISA-Geradensteigungen der Positivkontrollen (Kaninchen-anti-Pferd IgG-POD-Konjugat). Dies zeigt folgende Abbildung, in der E die Extinktion und t die Zeit bedeutet:

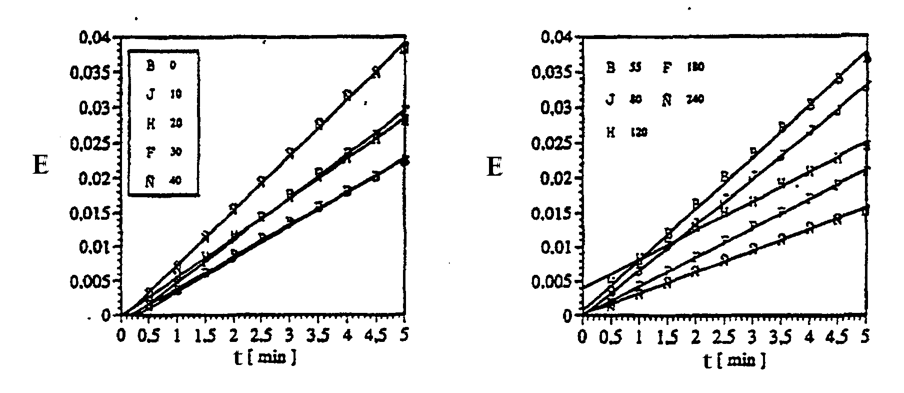

Berechnet man mit Hilfe der Eichmessung die daraus die aktiven Belegungsdichten, so erhält man die Abhängigkeit von der Bestrahlungsdauer.

Je länger bestrahlt wird, umso weniger Doppelbindungen verbleiben für die Generierung von Antikörper bindenden Aldehydfunktionen. Wäre dies der einzige Effekt, so müßte eine zunehmende Belichtungszeit eine abnehmende Belegungsdichte zur Folge haben. Also muß noch ein zweiter Effekt berücksichtigt werden. Die Höhe des von der Länge der Lichteinwirkung abhängigen Vernetzungsgrads bedingt die Stabilität des Films. Je länger also bestrahlt wurde, umso weniger Antikörper geht verloren, wenn sich nach der Immobilisierung (z. B. in den Waschschritten) Teile der Filmsubstanz mit darauf befindlichem IgG von der Glasoberfläche ablösen.

Die Gegenläufigkeit dieser Effekte erklärt das Maximum der Belegungsdichte.

Stabilität der Antikörperfilme auf Pcps:
Um die Haltbarkeit der immobilisierten Antikörper zu testen, wurden aktivierte hydrophobisierte Glasstückchen mit 10 LB-Monolagen Pcps III (25 mN/m) beschichtet, 55 Minuten bei 254 nm vernetzt, dann mit Osmiumtetroxid / Perjodat oxidiert, schließlich Ziege-anti-Kaninchen IgG immobilisiert und - nach der Blockierungsreaktion -in Phosphatpuffersalzlösung eingefroren. Nach verschiedenen Lagerzeiten wurden per ELISA-Test die aktiven Belegungsdichten bestimmt wobei in der Abbildung M = Menge an aktivem IgG und t = Zeit in Tagen bedentet :

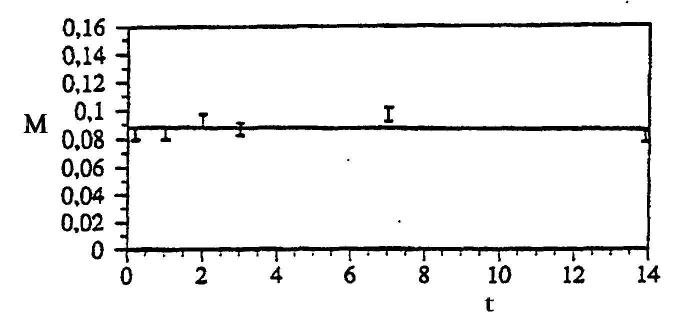

Die Aktivität der Antikörperfilme bleibt also für eine Dauer von zwei Wochen konstant. Die Filme sind offensichtlich durch die photochemische Vernetzung ausreichend stabilisiert worden. Eine Untersuchung der Stabilität an Luft ergibt dagegen einen drastischen Aktivitätsverlust innerhalb weniger Tage, da die Antikörper an Luft denaturiert werden.

### Beispiel 6

Vernetzung einer Monolage Pcps / Copolyglutamat:
Durch Arbeiten bei tieferen Temperaturen (15 °C: Anschluß eines Kryostaten an den LB-Trog) konnte der Kollapsdruck des Copolyglutamats auf 30 mN/m erhöht werden . Ferner bewirkte der Zusatz von 15 Masseprozent Pcps einen weiteren Anstieg auf insgesamt 33 mN/m. Dies ermöglichte den erneuten Versuch einer Vernetzung bei nunmehr 28 mN/m auszuführen. In der Tat wurden Folgende Ergebnisse beobachtet: die Filmfläche nahm während der Bestrahlung kontinuierlich ab und der Kollapsdruck entsprechend zu. Dem gemäß gelang die photochemische Vernetzung. Ein Grund dafür ist einerseits die durch die erhöhte Oberflächenspannung bewirkte Annäherung der olefinischen Doppelbindungen, wodurch sie eher vernetzbar werden. Andererseits wirkt Pcps als Sensibilisator, d. h. es dient zur Aufnahme des UV-Lichts, welches es dann auf das Copolyglutamat überträgt. Selbstverständlich können auch andere Sensibilisatoren hunzugegeben werden.

Der relativ geringe Beitrag der Vernetzung des Anteils an Peps dürfte innerhalb der Fehlergrenzen vernachlässigbar sein, so daß die registrierte Flächenabnahme und Oberflächenspannungszunahme in Abhängigkeit von der Bestrahlungsdauer auf eine photochemische Reaktion des Copolyglutamats zurückzuführen ist.

### Beispiel 7

Gemäß der Beispiele 1 bis 4 läßt sich also ein Beispiel für ein mögliches Immobilisierungskonzept wie folgt entwickeln: 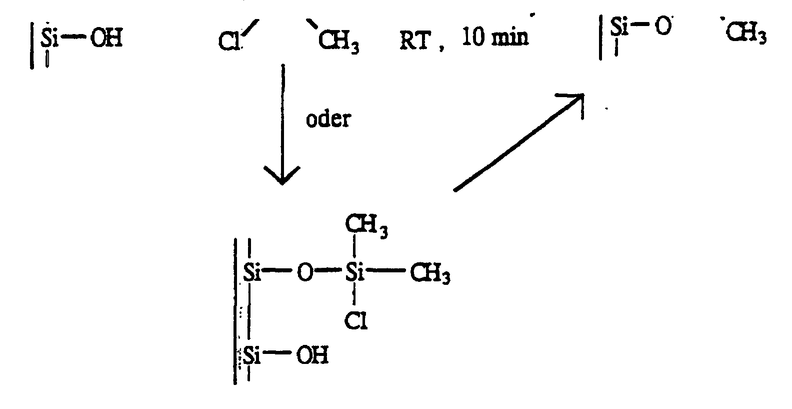
2 .) Beschichtung des hydrophobisierten Glasstückchens mit einem LB-Film
3.) Partielles "Anvernetzen" der am Film-Molekül befindlichen -terminalenolefinischen Doppelbindungen mit UV - Licht (254 nm)
4.) Oxidation von bisher unvernetzten Doppelbindungen zu Aldehydfunktionen: 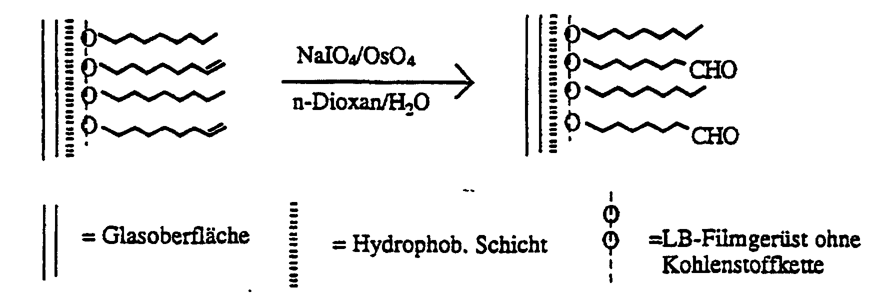
5.) Immobilisierung von Ziege-anti-Kaninchen IgG 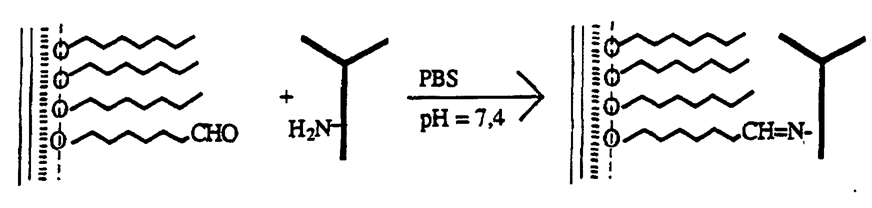
6.) Waschschritt: Entfernung von nicht immobilisiertem IgG
7.) Blockierung nicht abreagierter Aldehydfunktionen: (Umwandlung in Iminfunktionen durch Ethanolamin) 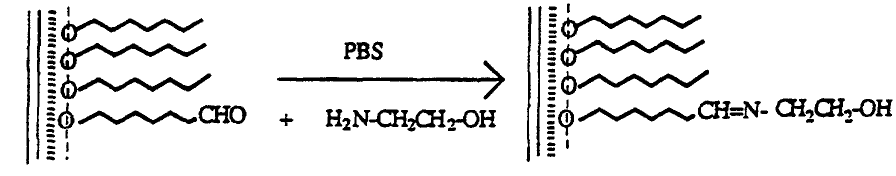
8.) ELISA:

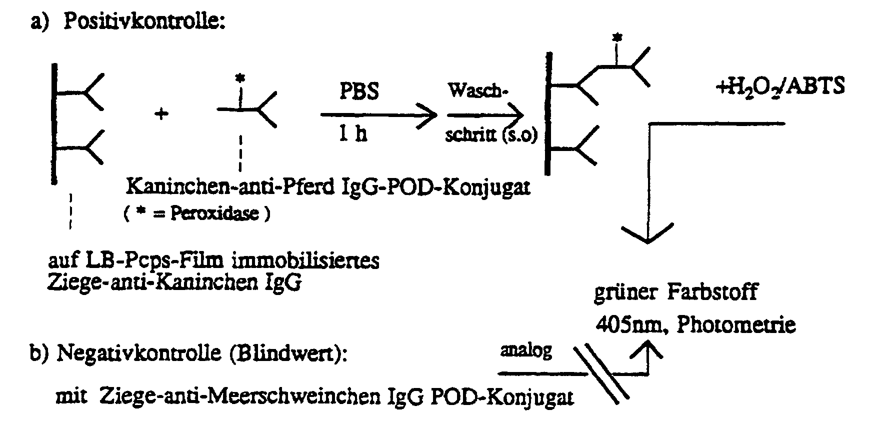

## Patentansprüche

1. Verfahren zur Immobilisierung von Biomolekülen auf oder in photochemisch vernetzbaren nicht-amphiphilen Langmuir-Blodgett-Filmen, **dadurch gekennzeichnet, daß** als Matrix eine nicht-amphiphile zwei- oder dreidimensional vernetzbare Substanz mit Hilfe der Langmuir-Blodgett-Technik
a) auf das Substrat aufgebracht wird und anschließend Rezeptor- oder Biomoleküle kovalent immobilisiert werden, oder
b) auf das Substrat aufgebracht wird, die Rezeptor- oder Biomoleküle jedoch zuvor mit der Matrixsubstanz cogespreitet wurden, oder
c) auf das Substrat aufgebracht wird, die Rezeptor- oder Biomoleküle jedoch so chemisch modifiziert wurden, daß sich ein an die Rezeptor- oder Biomoleküle angefügter weiterer Molekülteil derart in die Matrixsubstanz einlagert, daß die Rezeptor- oder Biomoleküle mit ihren bindungsfähigen Gruppen aus der Matrixsubstanz herausragen und zwar derart, daß sie ihre Aktivität noch besitzen.

2. Verfahren nach Anspruch 1, **dadurch gekennzeichnet, daß** die Matrix photochemisch vernetzbar ist.

3. Verfahren nach Anspruch 1 oder 2, **dadurch gekennzeichnet, daß** die Moleküle der Matrix funktionelle Gruppen an Seitengruppen aus der Gruppe Carbonyl-, Thionyl-, Imingruppen, C-C-Dreifachbindungen, bevorzugt C-C-Doppelbindungen besitzen, um Rezeptor- oder Biomoleküle zu binden.

4. Verfahren nach einem der Ansprüche 1 bis 3, **dadurch gekennzeichnet, daß** die Matrix aus mehreren unterschiedlichen nicht-amphiphilen Substanzen oder einer Mischung aus amphiphilen und nicht-amphiphilen Substanzen besteht.

5. Verfahren nach einem der Ansprüche 1 bis 4, **dadurch gekennzeichnet, daß** die als funktionelle Gruppen der Matrixmoleküle vorliegenden C-C-Doppelbindungen oxidativ nach der Art der Lemieux-Reaktion gespalten und damit auf dem Substrat zu Aldehydgruppen oxidiert werden, jedoch kein organisches Lösungsmittel zugesetzt wird.

6. Verfahren nach einem der Ansprüche 1 bis 5, **dadurch gekennzeichnet, daß** Rezeptor- oder Biomoleküle unterschiedlicher Bindungsspezifität in oder an ein und demselben Film gebunden sein können.

## Claims

1. Process for the immobilisation of biological molecules on or in photochemically crosslinkable non-amphiphilic Langmuir-Blodgett films, **characterised in that**, as the matrix, a non-amphiphilic two- or three-dimensionally crosslinkable substance, with the aid of the Langmuir-Blodgett technique, is
a) applied to the substrate, and receptor molecules or biological molecules are then covalently immobilised, or
b) applied to the substrate, but the receptor molecules or biological molecules have previously been co-spread with the matrix substance, or
c) applied to the substrate, but the receptor molecules or biological molecules have been chemically modified so that a further molecule part, added to the receptor molecules or biological molecules, is incorporated into the matrix substance in such a way that the bindable groups of the receptor molecules or biological molecules protrude from the matrix substance, specifically so that they still have their activity.

2. Process according to Claim 1, **characterised in that** the matrix is photochemically crosslinkable.

3. Process according to Claim 1 or 2, **characterised in that** the molecules of the matrix have functional groups on side groups from the group of carbonyl, thionyl, imine groups, C-C triple bonds, preferably C-C double bonds, in order to bind receptor molecules or biological molecules.

4. Process according to one of Claims 1 to 3, **characterised in that** the matrix consists of a plurality of different non-amphiphilic substances or a mixture of amphiphilic and non-amphiphilic substances.

5. Process according to one of Claims 1 to 4, **characterised in that** the C-C double bonds present as functional groups of the matrix molecules are cleaved oxidatively in the manner of the Lemieux reaction, and are thereby oxidised on the substrate to form aldehyde groups, but no organic solvent is supplied.

6. Process according to one of Claims 1 to 5, **characterised in that** receptor molecules or biological molecules with different binding specificity can be bound into or onto one and the same film.

## Revendications

1. Procédé d'immobilisation de molécules biologiques sur ou dans des films de Langmuir-Blodgett non amphiphiles réticulables par influences photochimiques, **caractérisé en ce que**, comme matrice, une substance non amphiphile bi- ou tridimensionnelle réticulable
a) est appliquée, à l'aide de la technique de Langmuir-Blodgett, sur le substrat et ensuite des molécules réceptrices ou biologiques sont immobilisées de manière covalente, ou
b) est appliquée sur le substrat, les molécules réceptrices ou biologiques ayant cependant été préalablement co-étalées avec la substance matrice, ou
c) est appliquée sur le substrat, les molécules réceptrices ou biologiques ayant cependant été modifiées chimiquement de façon à ce qu'une autre partie de molécule qui s'est ajoutée aux molécules réceptrices ou biologiques se dépose dans la substance matrice de façon à ce que les molécules réceptrices ou biologiques dépassent de la substance matrice avec leurs groupes possédant un pouvoir liant, et ce, de manière à ce qu'elles conservent leur activité.

2. Procédé selon la revendication 1, **caractérisé en ce que** la matrice est réticulable par influences photochimiques.

3. Procédé selon la revendication 1 ou 2, **caractérisé en ce que** les molécules de la matrice possèdent des groupes fonctionnels sur des groupes latéraux des groupes carbonyle, thionyle, imine, des liaisons triples C-C, de préférence des liaisons doubles C-C, pour lier les molécules réceptrices ou biologiques.

4. Procédé selon l'une quelconque des revendications 1 à 3, **caractérisé en ce que** la matrice est composée de plusieurs substances différentes non amphiphiles ou d'un mélange de substances amphiphiles et non amphiphiles.

5. Procédé selon l'une quelconque des revendications 1 à 4, **caractérisé en ce que** les doubles liaisons C-C présentes comme groupes fonctionnels des molécules de la matrice sont craquées par oxydation selon la réaction de Lemieux et ainsi oxydées en groupes aldéhydes sur le substrat, mais aucun solvant organique n'est ajouté.

6. Procédé selon l'une quelconque des revendications 1 à 5, **caractérisé en ce que** des molécules réceptrices ou biologiques ayant des spécificités de liaison différentes peuvent être liées dans ou sur un seul et même film.
